(19) Europäisches Patentamt / European Patent Office / Office européen des brevets

(11) **EP 4 484 473 A1**

(12) **EUROPEAN PATENT APPLICATION**
published in accordance with Art. 153(4) EPC

(43) Date of publication:
**01.01.2025  Bulletin 2025/01**

(21) Application number: **23818883.3**

(22) Date of filing: **11.05.2023**

(51) International Patent Classification (IPC):
*C08J 3/16* (2006.01)    *C08L 67/04* (2006.01)

(86) International application number:
**PCT/CN2023/093510**

(87) International publication number:
**WO 2023/236718 (14.12.2023 Gazette 2023/50)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **06.06.2022  CN 202210635146**

(71) Applicant: **Shanghai Bluepha Microbiology Technology Co., Ltd.**
**Shanghai 200072 (CN)**

(72) Inventors:
• **WANG, Dongsheng**
**Beijing 102206 (CN)**
• **MA, Wenyan**
**Beijing 102206 (CN)**
• **WU, Yakun**
**Beijing 102206 (CN)**
• **ZHANG, Jincheng**
**Beijing 102206 (CN)**
• **WANG, Jiepeng**
**Beijing 102206 (CN)**
• **SUN, Qiang**
**Beijing 102206 (CN)**
• **CAO, Yu**
**Beijing 102206 (CN)**

(74) Representative: **Michalski Hüttermann & Partner Patentanwälte mbB**
**Kaistraße 16A**
**40221 Düsseldorf (DE)**

(54) **PREPARATION METHOD FOR POLYHYDROXYALKANOATE AGGREGATE**

(57)    The present invention relates to the technical field of bioengineering, and specifically relates to a preparation method for a polyhydroxyalkanoate aggregate. The preparation method for a polyhydroxyalkanoate aggregate provided in the present invention comprises the step of making a polyhydroxyalkanoate suspension reach and maintain a turbulent flow state so as to agglomerate polyhydroxyalkanoate. By means of the preparation method of the present invention, a polyhydroxyalkanoate aggregate having a particle size of 50 µm or above can be prepared, and the prepared polyhydroxyalkanoate aggregate has relatively high purity and yield. The method has mild process conditions, simple steps and simple wastewater treatment, low cost, low requirements regarding equipment, and can realize large-scale industrial production.

**Description**

**Cross-reference to related application**

The present application claims priority to Chinese Patent Application No. 202210635146.8, entitled "preparation method for polyhydroxyalkanoate aggregate", filed on June 6, 2022, the entire disclosure of which is incorporated herein by reference in its entirety.

**Technical Field**

The present application relates to the field of bioengineering technology, in particular to a preparation method for a polyhydroxyalkanoate aggregate.

**Background Art**

Polyhydroxyalkanoate (hereinafter referred to as PHA) is a storable particle synthesized by microorganisms under conditions of other nutrient constraints while carbon sources surplus, and is a biopolymer polyester compound. PHA has the characteristics of common thermoplastics, but it differs from conventional petrochemical plastics. PHA has good biocompatibility, degradability, piezoelectricity, and optical activity, making it have broad application prospects in the field such as industry, agriculture, medicine and health, and food. PHA, as the best biodegradable material, has shown great potential in the field of degradable agricultural mulching films, degradable plastic products, and medicine.
Since PHA produced by microorganisms usually accumulates in the cell bodies of microorganisms in the form of inclusion body, it is necessary to separate and recover PHA particles inside cells. When PHA particles are used for plastic preparation, a good processing property is required, thus the purity of PHA particles is required to be high to minimize interference from impurities such as inorganic salts or cell lysates.
However, due to the complexity of microbial intracellular components, the extraction of PHA is quite difficult. In the long process of PHA development, researchers have conducted a lot of research on the extraction method thereof. The existing extraction processes include the organic solvent extraction method, mechanical crushing method, sodium hypochlorite-surfactant method, enzymatic method and the like. Among them, the disadvantages of the organic solvent method are that the solvent recovery is difficult, the production environment is dangerous, and it is only suitable for laboratory extraction. The mechanical crushing method mainly includes ultrasonic wall breaking or high-pressure homogenization wall breaking, which consumes a lot of energy and is difficult to scale up production. The disadvantages of the sodium hypochlorite-surfactant method include that sodium hypochlorite has strong irritation making the working environment poor, and seriously damages the molecular weight of PHA, and the wastewater containing surfactants is difficult to be treated. The disadvantages of the enzymatic method include that the use of a variety of expensive enzymes is required, and the cost is very high. Various problems existing in the above methods hinder the industrialized production of PHA.
PHA suspension is obtained by crushing microbial cells containing PHA. When recovering PHA particles from PHA suspension, the problem is that the PHA particles are too small to be filtered or centrifuged to obtain PHA particles. Although the prior art discloses some methods for increasing the particle size of PHA particles to facilitate separation, they all have different shortcomings as follows:

The invention with a publication number of EP1609868B1 discloses a method for collecting high-purity polyhydroxyalkanoates from microbial cells. The method includes:

performing the treatment of physical disruption and addition of low-temperature alkali in an aqueous suspension of microbial cells containing PHA to effectively disrupt the cells to recover PHA, then treating PHA with enzymes and/or surfactants, and then washing the PHA particles with a hydrophilic solvent for degreasing, deodorizing, and decolorizing, then aggregating the PHA particles by heating and stirring, followed by suction filtration and drying.
Although the reaction conditions are mild and the recovery rate is high, this method requires the use of a large amount of organic solvents to wash PHA particles, which leads to difficulties in downstream wastewater treatment, and explosion-proof workshops and explosion-proof equipment are required, which leads to difficulties in industrialized production.

The invention with a publication number of EP2357247B1 discloses a preparation method of poly-3-hydroxyalkanoate. The method includes: performing the treatment of physical disruption and addition of low-temperature alkali in an aqueous suspension of microbial cells containing PHA to effectively disrupt the cells to recover PHA, then treating PHA with enzymes and/or surfactants, and then washing the PHA particles with a large amount of water, adjusting the content of organic nitrogen in the aqueous suspension containing poly-3-hydroxyalkanoic acid to less than 1500 ppm per weight of poly-3-hydroxyalkanoic acid, the then aggregating PHA particles by heating and stirring, followed by suction filtration and drying. Although the reaction conditions are mild and the recovery rate is high, this method requires the use of a large amount of water to wash the particles by centrifugation. The preparation process generates a large amount of wastewater, which increases the difficulty of downstream wastewater treatment.

The invention with a publication number of EP3027761B1 discloses a method for recovering and purifying polyhydroxyalkanoate from cell culture, and the method for recovering and purifying PHA from cell culture includes: acidifying cell cultures and physically breaking cells to obtain PHA suspension. Subsequently, the PHA suspension was alkalized to a pH value equal to or higher than 8, and then diluted and subjected to tangential filtration. Then the PHA suspension was bleached, diluted, and tangentially filtered again, and then dried to obtain the finished product. This method can be carried out continuously without using organic solvents, and obtain PHA in a pure form without reducing the molecular weight. However, due to the small size of PHA particles, equipment blockage is prone to occur during tangential filtration, and calcium oxide, aluminum sulfate, phosphoric acid or their mixtures need to be added as flocculants to aggregate PHA particles to increase the particle size of PHA. Because the particle strength obtained by flocculants is not always very high, the metal salt and residual cell debris will contaminate the PHA polymer, reduce the purity of the finished PHA, and increase the difficulty of downstream material processing.

PHA particles are obtained by spray drying PHA suspension in the invention with a publication number of US9683076B2. However, spray drying consumes a lot of energy, and the residual inorganic salts and cell lysates in the suspension will be mixed in the PHA particles, which reduces the purity of the finished PHA and increases difficulties for downstream material processing.

To sum up, the methods disclosed in the prior art either require the use of a large amount of organic solvents or water for the washing of PHA particles, or require the use of flocculants which reduces the purity of finished products, or have problems such as complex processing procedure and high production costs.

## Summary

One purpose of the present application is to provide a preparation method for a polyhydroxyalkanoate aggregate and a polyhydroxyalkanoate aggregate prepared by the method.

To solve the problems that a large amount of organic solvent or water are needed to wash the polyhydroxyalkanoate particles so as to agglomerate in the method of aggregating polyhydroxyalkanoate to increase particle size, as well as high energy consumption and complex processes and the like, disclosed in the prior art, the present application has conducted extensive research on the process of polyhydroxyalkanoate aggregation. During the research and development process, the present application unexpectedly found that the turbulent flow state of the polyhydroxyalkanoate suspension is a key factor in promoting the aggregation of polyhydroxyalkanoate particles. It is difficult to achieve polyhydroxyalkanoate aggregation by stirring at low speed or even reaching the vortex state without washing the polyhydroxyalkanoate particles in the polyhydroxyalkanoate suspension. Once a turbulent flow state is reached, the polyhydroxyalkanoates in the polyhydroxyalkanoate suspension begin to aggregate rapidly and spontaneously, and the particle size increases significantly, and thus polyhydroxyalkanoate aggregates with a large particle size can be obtained in a short time. Although stirring is also involved in the aggregation method disclosed in the prior art, however, according to the aggregation process and results, the stirring used in these methods does not make the polyhydroxyalkanoate suspension reach a turbulent flow state, and the importance of the turbulent flow state for polyhydroxyalkanoate aggregation has never been discovered in the prior art.

Based on the above findings, the present application provides the following technical solutions:
The present application provides a preparation method for a polyhydroxyalkanoate aggregate, the method comprises the step of making a polyhydroxyalkanoate suspension reach and maintain a turbulent flow state so as to agglomerate the polyhydroxyalkanoate.

The turbulent flow state described above is a state when the Reynolds coefficient of the polyhydroxyalkanoate suspension is greater than the critical Reynolds coefficient of the polyhydroxyalkanoate suspension reaching the turbulence in the equipment in which it is located.

The above turbulent flow state can be carried out in a reactor or a pipe.

Taking a reactor as an example, the Reynolds coefficient in the reactor is calculated as follows:

$$Re_m = \frac{D^2 N \rho}{\mu}$$

wherein, D is the diameter of the turbine (m); N is the rotational speed of the agitator (r/s); $\rho$ is the density of the fluid (kg/m$^3$); and $\mu$ is the viscosity of the fluid (N.s/m$^2$).

When turbulent flow is carried out in the pipe, the Reynolds coefficient is calculated as: Re=$\rho$vd/$\mu$, wherein, v is the flow speed of the fluid (m/s), $\rho$ is the density of the fluid (kg/m$^3$), $\mu$ is the viscosity of the fluid (N.s/m$^2$), and d is a characteristic length (m).

Preferably, when the equipment where the polyhydroxyalkanoate suspension is located is a reactor, the turbulent flow state is a state of the suspension when the Reynolds coefficient of the polyhydroxyalkanoate suspension is greater than 1000.

When the equipment where the polyhydroxyalkanoate suspension is located is a pipe, the turbulent flow state is a state of the suspension when the Reynolds coefficient of the polyhydroxyalkanoate suspension is greater than 4000.

In some embodiments of the present application, the aggregation of polyhydroxyalkanoates is carried out in a reactor, and the turbulent flow state is that the Reynolds coefficient is greater than 1000, preferably greater than 1200, more preferably greater than 4000.

The present application has no specific limitation on the reactor, which can be any equipment that can make the material liquid in a turbulent flow state.

In some embodiments of the present application, the polyhydroxyalkanoate suspension is continuously stirred to achieve and maintain a turbulent flow state.

For polyhydroxyalkanoate suspension, it is preferred that the nitrogen content of the polyhydroxyalkanoate particles is less than 7000 ppm (relative to the polyhydroxyalkanoate per unit mass), and the nitrogen content of the suspension supernatant is less than 3000 ppm (relative to the suspension supernatant per unit mass).

The above-mentioned method for detecting the nitrogen content of polyhydroxyalkanoate particles and suspension supernatant is as follows: subjecting the polyhydroxyalkanoate suspension to centrifugation at 10000 rpm for 5 min, and the supernatant is the suspension supernatant; washing the resulted precipitate with water twice, and subjecting the resultant to centrifugation and drying to obtain polyhydroxyalkanoate particles; determining the nitrogen contents of the suspension supernatant and polyhydroxyalkanoate particles, respectively.

If nitrogen contents of polyhydroxyalkanoate particles and suspension supernatant are higher than the above target value, it will significantly inhibit the generation of polyhydroxyalkanoate aggregates, and even lead to no aggregates, or the impurity content of the finished product will be too high, and the purity of polyhydroxyalkanoate aggregates will be reduced.

The above-mentioned polyhydroxyalkanoate suspension is obtained by subjecting raw material of biological fermentation broth of polyhydroxyalkanoate to cell disruption and solid-liquid separation, and collecting polyhydroxyalkanoate precipitate, and then suspending by water.

The biological fermentation broth can be a fermentation broth containing an organism used for fermentation, which is produced by any organism capable of fermenting to produce polyhydroxyalkanoates.

For organisms, as long as they can accumulate polyhydroxyalkanoates in cells, they can be used without specific definitions. It is preferably microorganisms, such as *Aeromonas, Alcaligenes, Azotobacter, Bacillus, Clostridium, Halobacterium, Nocardia, Rhodospirillum, Pseudomonas, Ralstonia, Zoogloea.*

In particular, *Aeromonas* such as *Aeromonas caviae*; *Alcaligenes* such as *Alcaligenes lipolytica,* and *Alcaligenes latu*; and *Ralstonia* such as *Ralstonia eutropha.*

In the preparation process of the above polyhydroxyalkanoate suspension, there is no specific definition on the method of cell disruption, as long as the method can release the polyhydroxyalkanoate in the cell, such as chemical method, mechanical method, biological method.

The preparation method for aggregate of the present application has relatively simple requirements for the preparation process of the suspension, and the method of adjusting the fermentation broth to alkaline and then mechanically disrupting the cells can be used to release the polyhydroxyalkanoate particles in the cells.

In some embodiments of the present application, the pH of the fermentation broth is adjusted to alkaline (preferably 10.5 to 11.5) and placed for 1 to 3 h, and then the cells are disrupted by a mechanical method.

There is no specific definition on the method of solid-liquid separation, as long as the method can separate polyhydroxyalkanoate and non-polyhydroxyalkanoate. Methods such as centrifugation and filtration can be used, wherein, non-polyhydroxyalkanoate includes but is not limited to impurities such as cell debris, protein, nucleic acid.

The present application does not particularly define the concentration of polyhydroxyalkanoate in the polyhydroxyalkanoate suspension. It is preferred to use a higher concentration of polyhydroxyalkanoate suspension if the increased output of polyhydroxyalkanoate aggregate is desirable.

In some embodiments of the present application, it is preferred that the content of polyhydroxyalkanoate in the polyhydroxyalkanoate suspension is not less than 20 g/L. More preferably, it is more than 50 g/L.

Unlike the aggregation method of polyhydroxyalkanoates disclosed in the prior art, the present application has no specific definition on the pH of the polyhydroxyalkanoate suspension used for aggregation, wherein the polyhydroxyalkanoate suspension can be acidic, neutral, or alkaline.

If the method, specific as follows: adjusting the fermentation broth to alkaline, then disrupting the cells to release polyhydroxyalkanoate particles in the cells, collecting the polyhydroxyalkanoate precipitate through solid-liquid separation, and then suspending by water, is applied to prepare the polyhydroxyalkanoate suspension, there is no need to adjust the pH of the rehydrated suspension. At this time, the pH of the suspension is 8.5 to 10.5, The suspension can be directly used for the aggregation of polyhydroxyalkanoates. During the aggregation process of polyhydroxyalkanoates, keeping the temperature not less than 20°C is conducive to promoting the aggregation.

Preferably, during the process of maintaining the turbulent flow state, the temperature of the polyhydroxyalkanoate suspension is maintained at not less than 20°C.

In some embodiments of the present application, during the process of maintaining the turbulent flow state, the temperature of the polyhydroxyalkanoate suspension is maintained at not less than 30°C.

In some embodiments of the present application, during the process of maintaining the turbulent flow state, the temperature of the polyhydroxyalkanoate suspension is maintained at not less than 40°C.

Preferably, during the process of maintaining the turbulent flow state, the temperature of the polyhydroxyalkanoate suspension is maintained at 20°C to 70°C, more preferably 20°C to 40°C.

In the above-mentioned preparation method for aggregate, the time to maintain the turbulent flow state can be determined based on the particle size of the desired aggregate. The larger the particle size of polyhydroxyalkanoate aggregates, the longer the required maintenance time. In the process of aggregation, the particle size of the aggregate is monitored, and the aggregate is collected when the required particle size is reached. Particle size monitoring can be carried out by visual inspection or online real-time detection.

There is no specific definition on the particle size of the polyhydroxyalkanoate aggregates, as long as the particle size is suitable for industrial separation. It is generally preferred that the particle size is 5 $\mu$M or more, more preferably 50 $\mu$M or more, to be better suitable for industrial solid-liquid separation. In order to obtain polyhydroxyalkanoate aggregates suitable for solid-liquid separation, agglomerate until the polyhydroxyalkanoate aggregates with appropriate particle size are obtained, and then collect the polyhydroxyalkanoate aggregates through solid-liquid separation.

There is no specific definition on the methods of solid-liquid separation, including but not limited to centrifugation, filtration and the like.

In the above preparation method of aggregate, the aggregation of polyhydroxyalkanoate particles usually occurs within 0.1 to 3 h, and the particle size of polyhydroxyalkanoate aggregates increases significantly. It is preferred to maintain the turbulent flow state for 40 to 180 min.

According to the method of the present application, the average particle size of the polyhydroxyalkanoate aggregates is 10 $\mu$M or more, 50 $\mu$M or more, and even 100 $\mu$M or more. There is no specific definition on the upper limit of the particle size, but the average diameter is preferred to be 5000 $\mu$M or less, preferably 3000 $\mu$M or less.

In some embodiments of the present application, the preparation method for aggregate includes: subjecting polyhydroxyalkanoate suspension as a raw material to stirring to make the polyhydroxyalkanoate suspension reach and maintain a turbulent flow state, while maintaining the temperature not less than 20°C, to obtain the polyhydroxyalkanoate aggregates with increased particle size.

Further, in order to better remove impurities in the suspension and promote the formation of aggregates, the preparation method of the aggregates also includes the step of removing impurities in the polyhydroxyalkanoate suspension with enzymes.

The enzyme is used to treat impurities such as peptidoglycans, lipids, polysaccharides, nucleic acids attached to the surface of polyhydroxyalkanoate particles, including but not limited to one or more of protein decomposing enzymes, lipid decomposing enzymes, cell wall decomposing enzymes, and DNA decomposing enzymes. In specific applications, the above enzymes can be used alone or in combination of two or more. There are no specific definitions on the source of enzymes, which can be commercially available enzyme preparations or commercially available enzyme detergents for washing.

The additive amount of the enzyme depends on the type and activity of the enzyme. Although there is no specific definition, in order to reduce the cost, it is preferred to adopt an enzyme activity of 0.1 to 2 million U/mL and an enzyme addition amount of 0.001 mL/L-10 mL/L (relative to the polyhydroxyalkanoate suspension).

When adding enzymes to the aggregation system for impurity removal, the temperature and pH can be adjusted according to the optimal temperature and pH of the enzyme used, for example, the temperature is controlled at 20°C to 70°C and pH is 5 to 10. Since the optimal temperature and pH of enzymes are common knowledge among a person skilled in the art, they will not be described in detail in the present application.

Further preferably, to ensure the product quality of the final aggregate (mainly refers to the purity of the product) and make it better suitable for downstream material processing, the impurities in the polyhydroxyalkanoate suspension can also be removed with surfactants while adding enzymes, to further remove the impurities attached to the surface of PHA particles.

The surfactant mentioned above is one or more selected from anionic surfactant, cationic surfactant, amphoteric surfactant, and non-ionic surfactant, preferably anionic surfactant.

Although there is no need to limit the amount of surfactant, the preferred amount is 1 g/L-20 g/L (relative to polyhydroxyalkanoate suspension).

In some embodiments of the present application, the surfactant is SDS, and the amount of SDS is preferably 5-20 g/L.

In some embodiments of the present application, the surfactant is sodium dodecyl benzene sulfonate, and the amount of sodium dodecyl benzene sulfonate is preferably 2-10 g/L.

Unlike the method disclosed in the prior art, which first removing impurities with enzymes and/or surfactants, then washing and separating the polyhydroxyalkanoates, and then performing the aggregation of polyhydroxyalkanoates, the preparation method for aggregate of the present application can realize aggregation and impurity removal (impurity removal using enzymes and/or surfactants) in the same reaction system, greatly simplifies the preparation process, and at the same time, reduces the production of waste liquid.

Preferably, in the preparation method for aggregate, the above steps of removing impurities in the polyhydroxyalkanoate suspension are carried out in the same reaction system as the aggregation of polyhydroxyalkanoates.

The methods disclosed in the prior art mostly involve the use of organic solvent to wash the polyhydroxyalkanoate particles and then conducting the aggregation reaction, or conducting the aggregation reaction in the system containing organic solvent. The preparation method for aggregate of the present application can achieve the effective aggregation of polyhydroxyalkanoate without using organic solvent for washing and in the system not containing =organic solvent.

Preferably, the aggregation system of the polyhydroxyalkanoate does not contain organic solvents.

In some embodiments of the present application, the preparation method for a polyhydroxyalkanoate aggregate include: subjecting polyhydroxyalkanoate suspension as a raw material to stirring to make the polyhydroxyalkanoate suspension reach a turbulent flow state, adding enzymes, maintaining the turbulent flow state, and maintaining the temperature not less than 20°C, to obtain the polyhydroxyalkanoate aggregates with increased particle size. When reaching the target particle size, the polyhydroxyalkanoate aggregates were collected.

In some embodiments of the present application, the preparation method for a polyhydroxyalkanoate aggregate includes: subjecting polyhydroxyalkanoate suspension as a raw material to stirring to make the polyhydroxyalkanoate suspension reach a turbulent flow state, adding enzymes and surfactants, maintaining the turbulent flow state, and maintaining the temperature not less than 20°C, to obtain the polyhydroxyalkanoate aggregates with increased particle size. When reaching the target particle size, the polyhydroxyalkanoate aggregates were collected.

The collection of polyhydroxyalkanoate aggregates can be carried out by solid-liquid separation. With the increase in particle size of polyhydroxyalkanoate aggregates, the difficulty of solid-liquid separation of polyhydroxyalkanoate aggregates is significantly reduced, and the equipment cost can be reduced in industrial production. Therefore, there is no specific definition on solid-liquid separation methods and equipment, and the method of filtration (such as plate frame filter, basket bag filter) or centrifugation (such as horizontal screw centrifuge) can be used.

In order to remove impurities in the polyhydroxyalkanoate aggregate more thoroughly, it is preferred to wash the collected polyhydroxyalkanoate aggregates and then conduct solid-liquid separation to collect the washed polyhydroxyalkanoate aggregate.

In some embodiments of the present application, after washing the collected polyhydroxyalkanoate aggregates with water, the polyhydroxyalkanoate aggregate is collected through solid-liquid separation.

For the above polyhydroxyalkanoate aggregate, drying can also be carried out to prepare dry aggregate. The drying method is not particularly defined and can be room temperature ventilation drying, heating ventilation drying and the like.

Further, the present application provides polyhydroxyalkanoate aggregate prepared by adopting the preparation method for a polyhydroxyalkanoate aggregate described above.

Preferably, the particle size of the polyhydroxyalkanoate aggregate is at least 5 $\mu$M, more preferably at least 50 $\mu$M.

In some embodiments of the present application, the particle size of the polyhydroxyalkanoate aggregate is 50 to 100 $\mu$M.

In some embodiments of the present application, the particle size of the polyhydroxyalkanoate aggregate is 100 to 300 $\mu$M.

In the present application, the polyhydroxyalkanoate (PHA) can be various polyhydroxyalkanoate products, including but not limited to polyhydroxybutyric acid (PHB), poly (3 -hydroxybutyrate-co-3 -hydroxyvalerate) (PHBV), poly(3-hydroxybutyrate-co-3-hydroxycaproate) (PHBH) and poly(3-hydroxybutyrate-co-4-hydroxybutyrate) (P3HB4HB).

The beneficial effect of the present application at least lies in that the preparation method of the polyhydroxyalkanoate aggregate provided by the present application can produce polyhydroxyalkanoate aggregates with a particle size of 50 $\mu$M or more, which is suitable for industrial solid-liquid separation equipment, and the obtained polyhydroxyalkanoate aggregates have high purity and yield; and this method has mild process conditions, simple steps, simple wastewater treatment, low cost and low requirements for equipment, and can realize large-scale industrialized production.

Specifically, compared with the existing method of increasing the particle size of polyhydroxyalkanoate particles, the method of the present application has at least the following advantages:

1. in the preparation method for a polyhydroxyalkanoate aggregate provided by the present application, no organic solvent is used in the preparation process, the production environment is simple and safe, and there is no organic solvent residue in the prepared polyhydroxyalkanoate aggregate.

2. in the preparation method for a polyhydroxyalkanoate aggregate provided by the present application, no inorganic salt and no flocculants are needed in the preparation process, which effectively reduces the reagent cost of preparation.

3. in the preparation method for a polyhydroxyalkanoate aggregate provided by the present application, the efficient aggregation of polyhydroxyalkanoate can be achieved under low temperature conditions without relying on high-temperature operation, which effectively reduces energy consumption. The extraction equipment is simple and the cost is saved.

4. in the preparation method for a polyhydroxyalkanoate aggregate provided by the present application, there is no need to first wash the polyhydroxyalkanoate particles and then perform aggregation, the enzymatic decontamination process can be carried out in the same reaction system as the aggregation process, and there is no need to enzymatic remove impurity first and then perform aggregation, which effectively simplifies the preparation process and reduces the amount of wastewater.

## Specific Modes for Carrying Out the Embodiments

The following examples are intended to illustrate the present application, but are not intended to limit the scope of the present application.

In the following examples, the detection method of PHA purity is as follows:

30 mg of dried sample to be tested was taken, 2 mL of esterification solution and 2 mL of trichloromethane were added, and heated at 100°C for 4 h. After the reaction was completed, 1 mL of deionized water was added after the sample was cooled to room temperature, the resultant was subjected to vortex shaking, and standing for 30 to 60 min for layering. The upper layer was aqueous phase and the lower layer was organic phase; 1 mL of the lower organic phase was taken, a gas chromatograph (GC) (Shimadzu) for the detection of gas chromatography was used, and the purity of PHA was calculated. Wherein, the preparation method of esterification solution is as follows: 0.5 g of benzoic acid was weighed into a reagent bottle containing 485 mL methanol, 15 mL of concentrated sulfuric acid was slowly added into the methanol reagent bottle, and mixed well to complete the preparation of the esterification solution.

The nitrogen content was measured as follows: according to the method in 4.3 of GB/T-32019-2013, the nitrogen content was determined with a Kjeldahl nitrogen analyzer.

The particle size of PHA aggregates was detected as follows: the particle size was determined with the Truth Optics LT2100 laser particle size analyzer.

The PHA suspension used in the following examples was prepared as follows: after fermentation by PHA-producing strains, a fermentation broth containing 80% to 85% PHA with a biomass of 200 to 220 g/L was obtained; the pH of the fermentation broth was adjusted to 11, and maintained for 2 h, and the cells were disrupted by mechanical method. The resultant was subjected to centrifugation with a cup centrifuge at 10,000 rpm for 5 minutes, the supernatant was poured off, and the resultant was re-dissolved with water to the original volume to obtain a PHA suspension, wherein the PHA content was 163 g/L, the nitrogen content of PHA particles was 6530 ppm, and the nitrogen content in the supernatant was 2459 ppm.

## Example 1

The present Example provides a preparation method for a PHA aggregate. The steps of the method are as follows: the PHA suspension was added into a stirring reactor, the stirring was performed at 600 r/min, the temperature was raised to 40°C, SDS with a final concentration of 8 g/L and neutral protease with a final concentration of 1 mL/L were added, and the purification reaction was started. During the process, the particle size of PHA aggregate was detected every 10 min until the particle size did not continue to increase.

Wherein, the diameter of the turbine is 6 cm, the viscosity of the material liquid is 0.0075 N.s/m$^2$, and the density of the material liquid is 1030 kg/m$^3$. According to the Reynolds coefficient calculation formula, the Reynolds coefficient of material liquid in the reactor is 4944 after starting the stirring, achieving a sufficient turbulent flow state.

In the above methods, the change in particle size during the aggregation of PHA particles in the purification stage is shown in Table 1.

Table 1

| Purification time (min) | 0 | 10 | 20 | 30 | 40 | 50 | 60 |
|---|---|---|---|---|---|---|---|
| Particle size ($\mu$M) | 1.367 | 1.494 | 1.622 | 1.734 | 164.639 | 208.321 | 216.782 |

When the particle size of the PHA aggregates increased to 100 $\mu$M or above, suction filtration with a Buchner funnel was performed, and the obtained filter cake was rehydrated, washed twice, filtered, and dried at 80°C in an air blast drying oven. The purity of PHA is detected to be 99.1%, and the recovery rate is 98.5%.

**Example 2**

The present Example provides a preparation method for a PHA aggregate. The steps of the method are as follows: the PHA suspension was added into a stirring reactor, the stirring was performed at 150 r/min, the temperature was raised to 40°C, SDS with a final concentration of 8 g/L and neutral protease with a final concentration of 1 mL/L were added, and the purification reaction was started. During the process, the particle size of PHA aggregates was detected every 10 min until the particle size did not continue to increase.

Wherein, the diameter of the turbine is 6 cm, the viscosity of the material liquid is 0.0075 N.s/m$^2$, and the density of the material liquid is 1030 kg/m$^3$. According to the Reynolds coefficient calculation formula, the Reynolds coefficient of material liquid in the reactor is 1236 after starting the stirring, achieving a turbulent flow state.

In the above methods, the change in particle size during the aggregation of PHA particles in the purification stage is shown in Table 2.

Table 2

| Purification time (min) | 0 | 10 | 20 | 30 | 40 | 50 | 60 |
|---|---|---|---|---|---|---|---|
| Particle size ($\mu$M) | 1.373 | 1.368 | 1.345 | 1.379 | 159.775 | 204.854 | 205.324 |

When the particle size of the PHA aggregates increased to 100 $\mu$M or above, suction filtration with a Buchner funnel was performed, and the obtained filter cake was rehydrated, washed twice, filtered, and dried at 80°C in an air blast drying oven. The purity of PHA is detected to be 99.2%, and the recovery rate is 98.4%.

**Example 3**

The present Example provides a preparation method for a PHA aggregate. The steps of the method are as follows: the PHA suspension was added into a stirring reactor, the stirring was performed at 150 r/min, the temperature was raised to 40°C, SDS with a final concentration of 8 g/L and papain with a final concentration of 1 mL/L were added, and the purification reaction was started. During the process, the particle size of PHA aggregates was detected every 10 min until the particle size did not continue to increase.

Wherein, the diameter of the turbine is 6 cm, the viscosity of the material liquid is 0.0075 N.s/m$^2$, and the density of the material liquid is 1030 kg/m$^3$. According to the Reynolds coefficient calculation formula, the Reynolds coefficient of material liquid in the reactor is 1236 after starting the stirring, achieving a turbulent flow state.

In the above methods, the change in particle size during the aggregation of PHA particles in the purification stage is shown in Table 3.

Table 3

| Purification time (min) | 0 | 10 | 20 | 30 | 40 | 50 | 60 |
|---|---|---|---|---|---|---|---|
| Particle size ($\mu$M) | 1.334 | 1.358 | 1.353 | 1.356 | 138.733 | 177.235 | 179.431 |

When the particle size of the PHA aggregates increased to 100 $\mu$M or above, suction filtration with a Buchner funnel was performed, and the obtained filter cake was rehydrated, washed twice, filtered, and dried at 80°C in an air blast drying oven. The purity of PHA is detected to be 98.4%, and the recovery rate is 98.3%.

**Example 4**

The present Example provides a preparation method for a PHA aggregate. The steps of the method are as follows: the PHA suspension was added into a stirring reactor, the stirring was performed at 150 r/min, the temperature was raised to 40°C, SDS with a final concentration of 10 g/L and papain with a final concentration of 0.5 mL/L were added, and the purification reaction was started. During the process, the particle size of PHA aggregates was detected every 10 min until the particle size did not continue to increase.

Wherein, the diameter of the turbine is 6 cm, the viscosity of the material liquid is 0.0075 N.s/m$^2$, and the density of the material liquid is 1030 kg/m$^3$. According to the Reynolds coefficient calculation formula, the Reynolds coefficient of material liquid in the reactor is 1236 after starting the stirring, achieving a turbulent flow state.

In the above methods, the change in particle size during the aggregation of PHA particles in the purification stage is shown in Table 4.

Table 4

| Purification time (min) | 0 | 10 | 20 | 30 | 40 | 50 | 60 |
|---|---|---|---|---|---|---|---|
| Particle size ($\mu$M) | 1.326 | 1.328 | 1.245 | 1.349 | 146.378 | 153.894 | 162.432 |

When the particle size of the PHA aggregates increased to 100 $\mu$M or above, suction filtration with a Buchner funnel was performed, and the obtained filter cake was rehydrated, washed twice, filtered, and dried at 80°C in an air blast drying oven. The purity of PHA is detected to be 98.7%, and the recovery rate is 98.4%.

## Example 5

The present Example provides a preparation method for a PHA aggregate. The steps of the method are as follows: the PHA suspension was added into a stirring reactor, the stirring was performed at 150 r/min, the temperature was raised to 40°C, sodium dodecylbenzene sulfonate with a final concentration of 4 g/L, and neutral protease with a final concentration of 0.8 mL/L were added, and the purification reaction was started. During the process, the particle size of PHA aggregates was detected every 10 min until the particle size did not continue to increase.

Wherein, the diameter of the turbine is 6 cm, the viscosity of the material liquid is 0.0075 N.s/m$^2$, and the density of the material liquid is 1030 kg/m$^3$. According to the Reynolds coefficient calculation formula, the Reynolds coefficient of material liquid in the reactor is 1236 after starting the stirring, achieving a turbulent flow state.

In the above methods, the change in particle size during the aggregation of PHA particles in the purification stage is shown in Table 5.

Table 5

| Purification time (min) | 0 | 10 | 20 | 30 | 40 | 50 | 60 |
|---|---|---|---|---|---|---|---|
| Particle size ($\mu$M) | 1.324 | 1.358 | 1.328 | 1.461 | 144.746 | 149.785 | 157.789 |

When the particle size of the PHA aggregates increased to 100 $\mu$M or above, suction filtration with a Buchner funnel was performed, and the obtained filter cake was rehydrated, washed twice, filtered, and dried at 80°C in an air blast drying oven. The purity of PHA is detected to be 98.6%, and the recovery rate is 98.5%.

## Example 6

The present Example provides a preparation method for a PHA aggregate. The steps of the method are as follows: the PHA suspension was added into a stirring reactor, and the stirring was performed at 150 r/min, the temperature was 20°C, and the purification reaction was started. During the process, the particle size of PHA aggregates was detected every 10 min until the particle size did not continue to increase.

Wherein, the diameter of the turbine is 6 cm, the viscosity of the material liquid is 0.0075 N.s/m$^2$, and the density of the material liquid is 1030 kg/m$^3$. According to the Reynolds coefficient calculation formula, the Reynolds coefficient of material liquid in the reactor is 1236 after starting the stirring, achieving a turbulent flow state.

In the above methods, the change in particle size during the aggregation of PHA particles in the purification stage is shown in Table 6.

Table 6

| Purification time (min) | 0 | 30 | 60 | 90 | 120 | 150 | 180 |
|---|---|---|---|---|---|---|---|
| Particle size ($\mu$M) | 1.322 | 1.313 | 3.457 | 10.276 | 50.734 | 53.342 | 57.789 |

When the particle size of the PHA aggregates increased to 50 $\mu$M or above, suction filtration with a Buchner funnel was performed, and the obtained filter cake was rehydrated, washed twice, filtered, and dried at 80°C in an air blast drying oven. The purity of PHA is detected to be 94.6%, and the recovery rate is 87.3%.

**Example 7**

The present Example provides a preparation method for a PHA aggregate. The steps of the method are as follows: the PHA suspension was added into a stirring reactor, the stirring was performed at 600 r/min, the temperature was raised to 40°C, neutral protease with a final concentration of 1 mL/L was added, and the purification reaction was started. During the process, the particle size of PHA aggregates was detected every 10 min until the particle size did not continue to increase.

Wherein, the diameter of the turbine is 6 cm, the viscosity of the material liquid is 0.0075 N.s/m$^2$, and the density of the material liquid is 1030 kg/m$^3$. According to the Reynolds coefficient calculation formula, the Reynolds coefficient of material liquid in the reactor is 4944 after starting the stirring, achieving a sufficient turbulent flow state.

In the above methods, the change in particle size during the aggregation of PHA particles in the purification stage is shown in Table 7.

Table 7

| Purification time (min) | 0 | 10 | 20 | 30 | 40 | 50 | 60 |
|---|---|---|---|---|---|---|---|
| Particle size ($\mu$M) | 1.367 | 1.57 | 1.358 | 1.346 | 172.782 | 184.373 | 201.252 |

When the particle size of the PHA aggregates increased to 100 $\mu$M or above, suction filtration with a Buchner funnel was performed, and the obtained filter cake was rehydrated, washed twice, filtered, and dried at 80°C in an air blast drying oven. The purity of PHA is detected to be 97.1%, and the recovery rate is 98.3%.

**Example 8**

The present Example provides a preparation method for a PHA aggregate. The steps of the method are as follows: the PHA suspension used in Example 1 was diluted by adding 2 times the volume of water so that the content of PHA in the suspension was approximately 50 g/L, and the resultant was added into a stirring reactor, the stirring was performed at 600 r/min, the temperature was raised to 40°C, SDS with a final concentration of 8 g/L and neutral protease with a final concentration of 1 mL/L were added, and the purification reaction was started. During the process, the particle size of PHA aggregates was detected every 10 min until the particle size did not continue to increase.

Wherein, the diameter of the turbine is 6 cm, the viscosity of the material liquid is 0.0075 N.s/m$^2$, and the density of the material liquid is 1030 kg/m$^3$. According to the Reynolds coefficient calculation formula, the Reynolds coefficient of material liquid in the reactor is 4944 after starting the stirring, achieving a sufficient turbulent flow state.

In the above methods, the change in particle size during the aggregation of PHA particles in the purification stage is shown in Table 8.

Table 8

| Purification time (min) | 0 | 10 | 20 | 30 | 40 | 50 | 60 |
|---|---|---|---|---|---|---|---|
| Particle size ($\mu$M) | 1.321 | 1.294 | 1.452 | 1.368 | 1.743 | 54.321 | 58.754 |

When the particle size of the PHA aggregates increased to 50 $\mu$M or above, suction filtration with a Buchner funnel was performed, and the obtained filter cake was rehydrated, washed twice, filtered, and dried at 80°C in an air blast drying oven. The purity of PHA is detected to be 99.1%, and the recovery rate is 97.4%.

**Comparative Example 1**

The PHA suspension (the PHA suspension is the same as in Example 1) was added into a stirring reactor, the stirring was performed at 100 r/min, the temperature was raised to 40°C, SDS with a final concentration of 8 g/L and neutral protease with a final concentration of 1 mL/L were added, and the purification reaction was started. During the process, the particle size of PHA aggregates was detected every 10 min until the particle size did not continue to increase.

Wherein, the diameter of the turbine is 6 cm, the viscosity of the material liquid is 0.0075 N.s/m$^2$, and the density of the material liquid is 1030 kg/m$^3$. According to the Reynolds coefficient calculation formula, the Reynolds coefficient of material liquid in the reactor is 824 after starting the stirring, and a turbulent flow state is not achieved.

The change in particle size during the aggregation of PHA particles in the purification stage is shown in Table 9. The results showed that the particle size of the PHA did not increase significantly after purification for more than 3 h.

Table 9

| Purification time (min) | 0 | 30 | 60 | 90 | 120 | 150 | 180 |
|---|---|---|---|---|---|---|---|
| Particle size ($\mu$M) | 1.378 | 1.338 | 1.332 | 1.356 | 1.387 | 1.382 | 1.383 |

**Comparative Example 2**

The PHA suspension (the PHA suspension is the same as that in example 1) was added into the stirring reactor, and the stirring was performed at 100 r/min. The temperature was 20°C, and the purification reaction was started. During the process, the particle size of PHA aggregates was detected every 10 min until the particle size did not continue to increase. Wherein, the diameter of the turbine is 6 cm, the viscosity of the material liquid is $0.0075$ N.s/m$^2$, and the density of the material liquid is 1030 kg/m$^3$. According to the Reynolds coefficient calculation formula, the Reynolds coefficient of material liquid in the reactor is 824 after starting the stirring, and a turbulent flow state is not achieved.

The change in particle size during the aggregation of PHA particles in the purification stage is shown in Table 9. The results showed that the particle size of the PHA did not increase after purification for more than 3 h.

Table 10

| Purification time (min) | 0 | 30 | 60 | 90 | 120 | 150 | 180 |
|---|---|---|---|---|---|---|---|
| Particle size ($\mu$M) | 1.367 | 1.326 | 1.362 | 1.321 | 1.343 | 1.378 | 1.384 |

Although the present application has been described in detail with general descriptions and specific embodiments above, it is obvious to a person skilled in the art that some modifications or improvements can be made based on the present application. Therefore, these modifications or improvements made without deviating from the spirit of the present application all fall within the scope of protection claimed by the present application.

**Industrial Applicability**

The present application provides a preparation method for a polyhydroxyalkanoate aggregate. The preparation method for a polyhydroxyalkanoate aggregate provided by the present application comprises the step of making a polyhydroxyalkanoate suspension reach and maintain a turbulent flow state so as to agglomerate the polyhydroxyalkanoate. The preparation method of the present application can produce polyhydroxyalkanoate aggregates with a particle size of 50 $\mu$M or more, and the obtained polyhydroxyalkanoate aggregates have high purity and yield. This method has mild process conditions, simple steps, simple wastewater treatment, low cost, and low requirements for equipment, can realize large-scale industrialized production, and has good economic value and application prospects.

**Claims**

1.  A preparation method for a polyhydroxyalkanoate aggregate, wherein the method comprises the step of making a polyhydroxyalkanoate suspension reach and maintain a turbulent flow state so as to agglomerate polyhydroxyalkanoate.

2.  The preparation method for a polyhydroxyalkanoate aggregate according to claim 1, wherein the turbulent flow state is a state when the Reynolds coefficient of the polyhydroxyalkanoate suspension is greater than the critical Reynolds coefficient of the polyhydroxyalkanoate suspension reaching turbulence in the equipment in which it is located;

    preferably, the equipment is a reactor, and the turbulent flow state is a state when the Reynolds coefficient of the polyhydroxyalkanoate suspension is greater than 1000;
    alternatively, the equipment is a pipe, and the turbulent flow state is a state when the Reynolds coefficient of the polyhydroxyalkanoate suspension is greater than 4000.

3.  The preparation method for a polyhydroxyalkanoate aggregate according to claim 1 or 2, wherein in the polyhydroxyalkanoate suspension, the nitrogen content of the polyhydroxyalkanoate particles is less than 7000 ppm, and the nitrogen content of the suspension supernatant is less than 3000 ppm.

4. The preparation method for a polyhydroxyalkanoate aggregate according to claim 3, wherein the polyhydroxyalkanoate suspension is obtained by subjecting raw material of biological fermentation broth of polyhydroxyalkanoate to cell disruption and solid-liquid separation, and collecting the polyhydroxyalkanoate precipitate, and then suspending by water;
preferably, the content of polyhydroxyalkanoate in the polyhydroxyalkanoate suspension is not less than 20 g/L.

5. The preparation method for a polyhydroxyalkanoate aggregate according to any one of claims 1 to 4, wherein the temperature of the polyhydroxyalkanoate suspension is maintained at not less than 20°C in the process of maintaining the turbulent flow state.

6. The preparation method for a polyhydroxyalkanoate aggregate according to any one of claims 1 to 5, wherein the method further includes the step of removing impurities in the polyhydroxyalkanoate suspension with an enzyme;
preferably, the enzyme is one or more selected from protein decomposing enzyme, lipid decomposing enzyme, cell wall decomposing enzyme, and DNA decomposing enzyme.

7. The preparation method for a polyhydroxyalkanoate aggregate according to claim 6, wherein the method further includes the step of removing impurities in the polyhydroxyalkanoate suspension with a surfactant.

8. The preparation method for a polyhydroxyalkanoate aggregate according to claim 6 or 7, wherein the step of removing impurities in the polyhydroxyalkanoate suspension and the aggregation of polyhydroxyalkanoate are carried out in the same reaction system.

9. The preparation method for a polyhydroxyalkanoate aggregate according to any one of claims 1 to 8, wherein the reaction system of polyhydroxyalkanoate aggregation does not contain organic solvents.

10. A polyhydroxyalkanoate aggregate prepared by the preparation method for a polyhydroxyalkanoate aggregate according to any one of claims 1 to 9;
preferably, the polyhydroxyalkanoate aggregate has a particle size of at least 5 $\mu$M, more preferably at least 50 $\mu$M.

<div align="center">

**INTERNATIONAL SEARCH REPORT**

</div>

| International application No. |
|---|
| **PCT/CN2023/093510** |

| A. | CLASSIFICATION OF SUBJECT MATTER |
|---|---|

C08J3/16(2006.01)i;  C08L67/04(2006.01)i

According to International Patent Classification (IPC) or to both national classification and IPC

| B. | FIELDS SEARCHED |
|---|---|

Minimum documentation searched (classification system followed by classification symbols)

C08J, C08L, C08G, C12P

Documentation searched other than minimum documentation to the extent that such documents are included in the fields searched

Electronic data base consulted during the international search (name of data base and, where practicable, search terms used)

CNTXT, WPABSC, ENTXTC, VEN, CNKI, ISI, 百度学术, BAIDU SCHOLAR: 聚羟基脂肪酸酯, 聚-3-羟基链烷酸, PHA, 生物高分子聚酯, 凝聚体, 悬浮液, 湍流, 雷诺系数, 酶, 表面活性剂, 粒径, polyhydroxyalkanoate, poly-3-hydroxyalkanoic acid, biopolymeric polyester, aggregate, suspension, turbulent flow, Reynolds coefficient, enzyme, surfactant, particle size

| C. | DOCUMENTS CONSIDERED TO BE RELEVANT |
|---|---|

| Category* | Citation of document, with indication, where appropriate, of the relevant passages | Relevant to claim No. |
|---|---|---|
| PX | CN 116144046 A (BEIJING BLUEPHA MICROBIOLOGY TECHNOLOGY CO., LTD.) 23 May 2023 (2023-05-23)<br>claims 1-10 | 1-10 |
| X | CN 1694963 A (KANEKA CORP.) 09 November 2005 (2005-11-09)<br>description, p. 5, lines 7-8 and 16-29, p. 6, lines 6-31, p. 2, lines 6-11 | 1-6, 8-10 |
| Y | CN 1694963 A (KANEKA CORP.) 09 November 2005 (2005-11-09)<br>description, p. 5, lines 7-8 and 16-29, p. 6, lines 6-31, p. 2, lines 6-11 | 7-10 |
| Y | CN 111393625 A (COFCO NUTRITION AND HEALTH RESEARCH INSTITUTE CO., LTD. et al.) 10 July 2020 (2020-07-10)<br>description, paragraphs 4-11 | 7-10 |
| A | CN 111349218 A (JILIN COFCO BIO-CHEMICAL CO., LTD. et al.) 30 June 2020 (2020-06-30)<br>description, paragraphs 10, 14, 19, 22-23, and 27 | 1-10 |
| A | JP 2019041606 A (KANEKA CORP.) 22 March 2019 (2019-03-22)<br>description, embodiment 1 | 1-10 |

☑ Further documents are listed in the continuation of Box C.     ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **10 July 2023** | **08 August 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

<table>
<tr><td colspan="2" align="center"><strong>INTERNATIONAL SEARCH REPORT</strong></td><td>International application No.<br><strong>PCT/CN2023/093510</strong></td></tr>
</table>

**C.    DOCUMENTS CONSIDERED TO BE RELEVANT**

| Category* | Citation of document, with indication, where appropriate, of the relevant passages | Relevant to claim No. |
|---|---|---|
| A | WO 2010116681 A1 (KANEKA CORP. et al.) 14 October 2010 (2010-10-14)<br>description, paragraphs 0032-0033, and embodiments 1-2 | 1-10 |

International application No.

**PCT/CN2023/093510**

| Patent document cited in search report | | | Publication date (day/month/year) | Patent family member(s) | | | Publication date (day/month/year) |
|---|---|---|---|---|---|---|---|
| CN | 116144046 | A | 23 May 2023 | None | | | |
| CN | 1694963 | A | 09 November 2005 | WO | 2004033700 | A1 | 22 April 2004 |
| | | | | AU | 2003266709 | A1 | 04 May 2004 |
| | | | | CA | 2499607 | A1 | 22 April 2004 |
| | | | | JPWO | 2004033700 | A1 | 09 February 2006 |
| | | | | BR | 0314749 | A | 26 July 2005 |
| | | | | EP | 1550724 | A1 | 06 July 2005 |
| | | | | PL | 376044 | A1 | 12 December 2005 |
| | | | | RU | 2005113285 | A | 20 January 2006 |
| | | | | US | 2008118963 | A1 | 22 May 2008 |
| CN | 111393625 | A | 10 July 2020 | CN | 111393625 | B | 01 September 2020 |
| CN | 111349218 | A | 30 June 2020 | US | 2021340316 | A1 | 04 November 2021 |
| | | | | US | 11203663 | B2 | 21 December 2021 |
| | | | | CN | 111349218 | B | 09 February 2021 |
| JP | 2019041606 | A | 22 March 2019 | JP | 6864585 | B2 | 28 April 2021 |
| WO | 2010116681 | A1 | 14 October 2010 | JP | 2012115145 | A | 21 June 2012 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202210635146
- EP 1609868 B1
- EP 2357247 B1
- EP 3027761 B1
- US 9683076 B2